(19) Europäisches Patentamt
European Patent Office
Office européen des brevets

(11) Publication number: **0 113 996 A2**

# (12) EUROPEAN PATENT APPLICATION

(21) Application number: 83307797.7

(22) Date of filing: 21.12.83

(51) Int. Cl.[3]: **C 07 C 103/52**, C 12 Q 1/38

(30) Priority: 21.12.82 JP 224684/82

(43) Date of publication of application: 25.07.84 Bulletin 84/30

(84) Designated Contracting States: CH DE FR GB IT LI

(71) Applicant: **TAISHO PHARMACEUTICAL CO. LTD, 24-1 Takata 3-chome Toshima-ku, Tokyo 171 (JP)**

(72) Inventor: **Tamai, Masaharu, 6-18 Ayase Hasuda-shi, Saitama-ken (JP)**
Inventor: **Adachi, Takashi, 1-1205 Aoba Kuki-shi, Saitama-ken (JP)**
Inventor: **Oguma, Kiyoshi, 5-16-8 Mochida Gyodashi, Saitama-ken (JP)**
Inventor: **Hanada, Kazunori, 523-12 Haraichi Ageo-shi, Saitama-ken (JP)**
Inventor: **Omura, Sadafumi, 523-7 Haraichi Ageo-shi, Saitama-ken (JP)**
Inventor: **Katunuma, Nobuhiko, 3-246-2 Myodo-cho Tokushima-shi, Tokushima-ken (JP)**

(74) Representative: **Ruffles, Graham Keith et al, MARKS & CLERK 57-60 Lincoln's Inn Fields, London WC2A 3LS (GB)**

## (54) Peptide derivatives.

(57) Novel peptide derivatives useful as substrate for assay of EC 3.4.22 enzymes, for example, cathepsin L, cathepsin B and cathepsin H, contain an L-methionine radical and are of the formula (I)

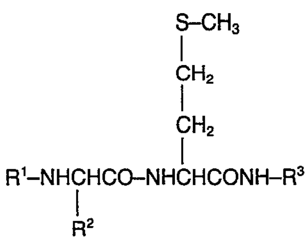

wherein $R^1$ represents an aliphatic acyl group containing 2–5 carbon atoms which is substituted with one or two methyl, amino, carboxyl hydroxy or benzyloxy groups, a tertiary butoxycarbonyl group or an aminobenzoyl group; or a hydrogen atom; $R^2$ represents a 2-methylpropyl, 1-methylpropyl, p-hydroxybenzyl, p-methoxybenzyl, benzyl or 3-guanidylpropyl group; and $R^3$ represents a naphthyl, methylcoumaryl or nitrophenyl group; or an acid addition salt thereof.

EP 0 113 996 A2

M&C FOLIO: 799P47389 WANGDOC: 0252C

PEPTIDE DERIVATIVES

The present invention relates to novel peptide derivatives of use as substrates for measuring protease activities.

More particularly, this invention is concerned with peptide derivatives useful as colour-developing or fluorescent substrates for measuring the activity of cystein proteases in which a thiol group is essential for the exertion of the activity.

Cysteine proteases in which a thiol group is essential for activity comprise those classified in the group EC 3.4.22 (as classified by the Recommendations of the Nomenclature Committee of the International Union of Biochemistry on the Nomenclature and Classification of Enzymes, in "Enzyme Nomenclature", published by Academic Press, 1979). These enzymes are present in mammalian tissues such as muscles and liver and play an important physiological role. Measurement of the activity of these enzymes is significant for elucidating many physiological actions in mammal tissues and is applicable to the diagnosis of disease.

The activity of enzymes of the group EC 3.4.22, for example cathepsin B [EC 3.4.22.1], cathepsin L [EC 3.4.22.-] and cathepsin H [EC 3.44.22.-], has been assayed using BANA ($\alpha$-N-benzoyl-DL-arginine-2-naphthylamide) as substrate. BANA, however, is of low sensitivity for enzymes of the group EC 3.4.22, and it has been difficult to effect the BANA assay when the specimen to be assayed contains only a small amount of the enzyme. Particularly for muscle specimens which do contain a very small amount of the enzyme, substrates of high sensitivity are needed.

It is therefore an object of the present invention to provide substrates which enable the assay of enzymes, including enzymes of the group EC 3.4.22, using simple procedures and at a high sensitivity.

We made extensive investigation to find substrates which made possible a simple and sensitive measuremet for the activity of enzymes of the group EC 3.4.22 present in mammallan tissues. We have now achieved synthesis of certain L-methione-containing peptide derivatives and found that such derivatives can be a colour- developing or fluorescent substrate which enables a simple and sensitive assay of enzymes of the group EC 3.4.22.

According to the invention, there are provided peptide derivatives havng the general formula:

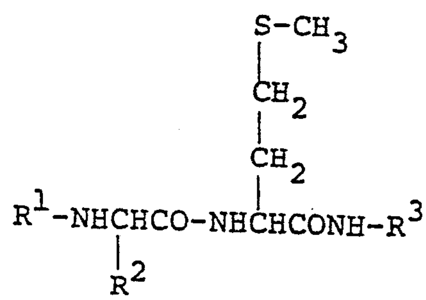

(I)

wherein $R^1$ represents an aliphatic acyl group containing 2-5 carbon atoms substituted with one or two methyl, amino, carboxy, hydroxy or benzyloxy groups; a tertiary butoxycarbonyl group; an aminobenzoyl group; or a hydrogen atom ; $R^2$ represents a 2-methylpropyl, 1-methylpropyl, p-hydroxybenzyl, p-methoxybenzyl, benzyl or 3-guanidylpropyl group; and $R^3$ represents a naphthyl, methylcoumaryl or nitrophenyl group; and acid addition salts thereof.

The peptide derivatives [I] are hydrolyzed by the action of an enzyme of the group EC 3.4.22 to release a colour-forming or fluorescent substrate $R^3H$. By photometrically measuring the concentration of the substance, the activity of the enzyme can be determined. The above hydrolysis reaction is so sensitive that highly sensitive assay of the

abovementioned enzymes is feasible with the peptide derivatives [I].

Preferred peptide derivatives [I] are the compounds wherein $R^1$ is a succinyl, leucyl or ß-alanyl group, $R^2$ is a p-hydroxybenzyl group and $R^3$ is a naphthyl or p-nitrophenyl group.

Particularly preferred peptide derivatives [I] are D-leucyl-L-tyrosyl-L-methionine-ß-naphthylamide, ß-alanyl-L-tyrosyl-L-methionine-ß-naphthylamide, glycoloyl-L-tyrosyl-L-methionine-ß-naphthylamide, succinyl-L-tyrosyl-L-methionine-ß-naphthylamide, or succinyl-L-tyrosyl-L-methionine-p- nitroanilide.

The peptide derivatives [I] of the invention can be prepared by methods conventionally employed in peptide chemistry. Examples of such methods include the following:

<u>Method A</u>

(1) A coupling method which comprises condensing a compound having the general formula

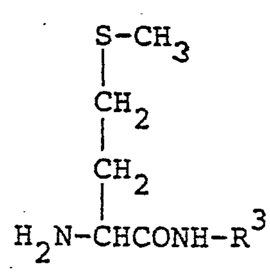

[II]

(wherein $R^3$ is as defined above) with a compound having the general formula

$$R^4\text{-NH}\underset{\displaystyle R^2}{\underset{|}{\text{C}}}\text{HCO-OH}$$

[III]

(wherein $R^2$ is as defined above and $R^4$ is as $R^1$ defined above or represents an amino protecting group) in the presence of a condensing agent such as N,N'-dicyclohexylcarbodiimide or 1-ethyl-3-(3'-dimethylaminopropyl)carbodiimide, to give a compound (VII).

When $R^4$ in the compounds [III] is a protecting group, it can be one conventionally employed in peptide chemistry. Typical examples are benzyloxycarbonyl, p-methoxybenzyloxycarbonyl, tert.-butoxycarbonyl, tert.-amyloxycarbonyl, o-nitrophenylsulphenyl, trityl and like groups.

After the condensation, the protecting group in compound [VII] can then be removed to produce a compound [I] wherein $R^1$ is a hydrogen atom. Removal can be effected using a cleavage reaction conventional in peptide chemistry. For example, when the $R^4$ is a tert.-butoxycarbonyl group the removal can be accomplished by an acid treatment, and when the group $R^4$ is a benzyloxycarbonyl or p-methoxybenzylcarbonyl

group, it can be removed by a catalytic reduction or an acid treatment.

The condensation reaction is preferably carried out by stirring a mixture of the compound [II], the compound [III] and the condensing agent in a solvent such as tetrahydrofuran, dimethylformamide, chloroform, ethyl acetate or dichloromethane at room temperature or in an ice-bath for from 3 hours to overnight.

(2) As a variation of method A (1), a mixed acid anhydride method comprises converting the compound [III] to a mixed acid anhydride with a chloroformic acid ester such as isobutyroxycarbonyl chloride or ethyloxycarbonyl chloride and then reacting the mixed acid anhydride with the compound [II].

The mixed acid anhydride can be prepared by reacting the compound [III] and the acid chloride in the presence of a solvent such as tetrahydrofuran, ethyl acetate, dimethylformamide or dimetyl sulfoxide at a temperature of -15 to -10°C for a period of 2 to 20 minutes.

The reaction of the mixed acid anhydride with the compound [II] is preferably carried out in the presence of a solvent such as tetrahydrofuran, ethyl acetate, chloroform, dimethyl sulphoxide or dimethylformamide, at

a temperature of -15 to -10°C for about 1 hour and then at room temperature for from 1 hour to overnight.

(3) As a further variation, a method comprises reacting the compound [III] with a hydroxy compound such as N-hydroxysuccinimide or p-nitrophenol to prepare an active ester and then reacting the active ester with the compound [II].

The active ester can be prepared by reacting the compound [III] and the hydroxy compound in the presence of a solvent such as tetrahydrofuran, dioxane, chloroform, dimethylformamide, dichloromethane or ethyl acetate while cooling in an ice-bath or while at room temperature for 2 hours to overnight.

The reaction of the active ester and the compound [II] can then be carried out in a solvent under the same conditions as in the aforementioned step.

Method B

(1) This method comprises combining a peptide derivative [I] wherein $R^1$ is hydrogen atom with a compound having the general formula

$$R^5-OH \qquad (IV)$$

wherein $R^5$ is as defined for $R^1$ except for a hydrogen atom, or with a compound [IV] protected with an appropriate protecting group. Protection can be used when $R^7$ is an aliphatic acyl group substituted with amino, carboxyl or hydroxy, or when $R^1$ is an aminobenzoyl group. If a protecting group is used, it can subsequently be removed by an appropriate method to prepare the desired peptide derivative.

(2) An example of Method B(1) for compounds (1) where $R^1$ is acyl substituted with one carboxy group comprises reacting a peptide derivative [I] wherein $R^1$ is a hydrogen atom with a dicarboxylic anhydride such as malonic, succinic or glutaric anhydride to form a peptide derivative [I] wherein $R^1$ is an acyl substituted with a carboxy group. Such a method is carried out by reacting the peptide derivative [I] wherein $R^1$ is a hydrogen atom with the dicarboxylic anhydride in the presence of a solvent such as ethyl ether, acetonitrile, tetrahydrofuran or chloroform with cooling with ice or at room temperature for from 2 hours to overnight.

<u>Method C</u>

This method comprises reacting a compound having the general formula

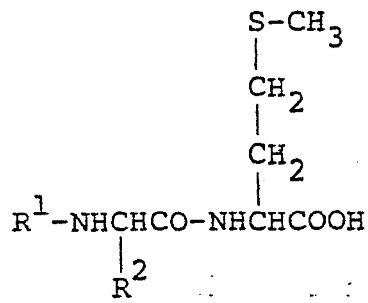

[V]

wherein $R^1$ and $R^2$ are as defined above and protected as necessary, with a compound having the general formula

$$H_2N-R^3 \qquad [VI]$$

wherein $R^3$ is as defined above. A procedure analogous to the above-described method A (2) or (3) can be used, and any protecting group can then be removed by an appropriate method to give the peptide derivative [I]. The same protecting groups as described in the method A may be employed.

The compounds [II] to [VI] employed in the methods A to C are known or similar to known compounds and may be prepared by conventional methods.

The peptide derivatives of the present invention may be converted to acid addition salts by the conventional methods, if desired. Examples of the acid for forming

the acid addition salts are formic, trifluoroacetic, hydrochloric and sulphuric acids.

The peptide derivatives [I] of the invention are useful as a colour-developing or fluorescent substrate for the assay of cysteine proteinases in which a thiol group is essential for the exertion of the activity.

In employing the peptide derivatives [I] of the invention as a substrate for the proteases of the group EC 3.4.22, the enzyme can be incubated with a predetermined amount of the peptide derivative [I] in an appropriate buffer solution at a predetermained temperature for a predetermined period of time. By measuring the naphthylamine, methylaminocoumarin or nitroaniline thus liberated, the enzymatic activity can be determined at a high sensitivity.

For preference, the liberated compound is ß-naphthylamine, 4-methyl-7-aminocoumarin or p-nitroanine. Various methods are available in particular for the measurement of ß-naphthylamine, 4-methyl-7-aminocoumarin or p-nitroaniline. When ß-naphthylamine is liberated, excitaton is effected at around 355 nm and the intensity of the fluoroescence around 410 nm measured with a flurometer, or a colour can be develoed with Fast Garnet GBC Salt and absorbance around 520 nm measured with a spectrophotometer. For

4-ethyl-7-aminocoumarin, an excitation is effected at around 380 nm and the intensity of the fluorescence at 440 nm measured with a fluorometer. For p-nitroaniline, measurement of the absorbance around 410 nm is preferable because it is simple and sensitive.

The invention wil be described in examples which comprise tests illustrating that the peptide derivatives [I] of the invention are highly sensitive substrates for proteinases as well as examples of the preparation of the peptide derivatives [I].

Test Example 1

Pure preparations of cathepsins B, L and H from rat livers were used according to the method of A J Barrett (Analytical Biochemistry

vol. 47, pp. 280 - 293 (1972)] except that various peptide derivatives of the general formula [I] were used in place of N-benzoyl-alginine-β-naphthylamide, and the reactions were carried out for 20 min. at pH 5.0.

To 2 mℓ. of a mixture containing ethylenediaminetetraacetic acid (1 mM), cysteine (2 mM), acetate buffer (pH 5.0, 0.1 M) and an enzyme preincubated at 40°C. for 5 min. was added 50 μℓ. of a dimethyl sulfoxide solution of a peptide derivative of the general formula [I] (80 mM). The resulting mixture was incubated at 37°C. for 20 min. To the reaction mixture was added 2 mℓ. of a solution containing p-chlormercuribenzoic acid (5 mM), ethylenediaminetetraacetic acid (25 mM), Brij-35 (0.2 %) and Fast Garnet GBC salt (250 μg./mℓ.) After several minutes was added 4 mℓ. of n-butanol. The mixture was vigorously shaken and centrifuged. The organic layer was separated, for which absorbance at 520 nm was measured.

When the liverated substance was β-naphthylamine, measurement was made according to the above-cited method of A. J. Barrett.

The liberated amount was determined for 4-methyl-7-aminocoumarin by exciting at 380 nm and measuring intensity of the fluorescence at 440 nm with a fluorometer, and for p-nitroaniline by measuring absorbance at 410 nm with a spectrophotometer.

Hydrolyzability of the peptide derivatives [I] with the above-cited cathepsins was expressed in terms of μ mols of β-naphthylamine, 4-methyl-7-aminocoumarin or p-nitroaniline liberated in one minutes per mg. of the enzyme. Results are shown in Table 1.

Table 1. Hydrolyzability of substrates.

| Compound No. | Cathepsin L | Cathepsin B | Cathepsin H |
|---|---|---|---|
| 1 | 0.701 | 0.315 | 0.012 |
| 2 | 0.473 | 0.043 | 0.035 |
| 3 | 1.198 | 0.341 | 0.023 |
| 4 | 1.252 | 0.266 | 0.184 |
| 5 | 0.139 | 0.090 | 1.209 |
| 6 | 0.702 | 0.180 | 0.023 |
| 7 | 0.400 | 1.310 | 0.155 |
| 8 | 2.491 | 2.981 | 0.009 |
| 9 | 2.106 | 2.486 | 0 |
| 10 | 0.950 | 2.396 | 0.029 |
| 11 | 1.565 | 2.246 | 0 |
| 12 | 0.722 | 0.530 | 0 |
| 13 | 0.343 | 0.826 | 0 |
| 15 | 0.080 | 0.006 | 0 |
| 17 | 1.637 | 1.120 | 0 |

Note. Compound number is referred to by the example number in which the compound was prepared.

Test Example 2.

The same procedures as those in Test Example 1 above were followed except that phosphate buffer (pH 6.0, 0.1 M) was used in place of the acetate buff (pH 5.0, 0.1 M) to determine hydrolyzability of the peptide derivatives [I]. Results are shown in Table 2.

Table 2. Hydrolyzability of substrates.

| Compound No. | Cathepsin L | Cathepsin B | Cathepsin H |
|---|---|---|---|
| BANA | 0.117 | 0.669 | 1.884 |
| 8 | 7.583 | 1.246 | 0.020 |
| 9 | 7.582 | 1.901 | 0.026 |
| 17 | 5.107 | 1.925 | 0.068 |

Example 1.

To a solution of 1.0 g. of tert.-butoxycarbonyl-L-tyrosin, 0.98 g. of L-methionine-β-naphthylamide, 0.53 g. of 1-hydroxybenzotriazole and 0.39 g. of N-methylmorpholine in 80 mℓ. of tetrahydrofuran was added 0.75 g. of 1-ethyl-3-(3-dimethylaminopropyl)carbodiimide slowly

with stirring and ice cooling. The mixture was stirred with ice cooling for 2 hours and at room temperature for additional 2 hours, followed by concentration under reduced pressure. To the concentrate were added 100 mℓ. of ethyl acetate and 80 mℓ. of water. The mixture was vigorously shaken, and the ethyl acetate layer was separated, which was successively washed with 10 % citric acid solution, saturated aqueous sodium bicarbonate and saturated aqueous sodium chloride and then concentrated to dryness. The residue thus obtained was crystallized from ethyl acetate-n-hexane. There was obtained 1.31 g. of tert.-butoxycarbonyl-L-tyrosyl-L-methionine-β-naphthylamide. Yield 71 %. m.p. 201 - 202°C.

Example 2.

A solution of 523 mg. of tert.-butoxycarbonyl-L-tyrosyl-L-methionine-β-naphthylamide in 10 mℓ. of formic acid was stirred at room temperature for 3 hours and then concentrated under reduced pressure. The residue thus obtained was crystallized from ethyl acetate-n-hexane to give 447 mg. of L-tyrosyl-L-methionine-β-naphthylamine formate. Yield 95 %. m.p. 175 - 177°C.

Example 3.

A solution of 500 mg. of tert.-butoxycarbonyl-L-phenylalanyl-L-methionine-β-naphthylamine which had been prepared in the same way as in Example 1 from tert.-butoxycarbonyl-L-phenylalanine and L-methionine-β-naphthylamide in 10 mℓ. of formic acid was treated in the same way as in Example 2 to obtain 335 mg. of L-phenylalanyl-L-methionine-β-naphthylamide formate. Yield 83 %. m.p. 142 - 144°C.

Example 4.

A solution of 974 mg. of tert.-butoxycarbonyl-L-leucyl-L-methionine-β-naphthylamide which had been prepared in the same way as in Example 1 from tert.-butoxycarbonyl-L-leucine monohydrate and L-methionine-β-naphthylamide in 30 mℓ. of formic acid was treated in the same way as in Example 2 to obtain 675 mg. of L-leucyl-L-methionine-β-naphthylamide formate. Yield 78 %. m.p. 118 - 119°C.

Example 5.

A solution of 600 mg. of tert.-butoxycarbonyl-L-isoleucyl-L-methionine-β-naphthylamide which had been obtained in the same way as in Example 1 from tert.-butoxycarbonyl-L-isoleucine and L-methionine-β-naphthylamide in 20 mℓ. of formic acid was treated in the same way as in Example 2 to obtain 456 mg. of L-isoleucyl-L-methionine-β-naphthylamide formate. Yield 85.5 %. m.p. 156 - 157°C.

Example 6.

Into a solution of 250 mg. of *tert*.-butoxycarbonyl-L-tyrosyl-L-methionine-β-naphthylamide in a mixed solvent of methanol-ether-dichloromethane was bubbled diazomethane. The resulting mixture was allowed to stand overnight at room temperature, to which was then added acetic acid. The mixture was concentrated under reduced pressure, and the residue was crystallized from ethyl acetate-*n*-hexane. There was obtained 200 mg. of *tert*.-butoxycarbonyl-O-methyl-L-tyroxyl-L-methionine-β-naphthylamide, which was dissolved in 10 mℓ. of formic acid. The solution was treated in the same way as in Example 2 to obtain 148 mg. of O-methyl-L-tyrosyl-L-methionine-β-naphthylamide formate. Yield 82.2 %. m.p. 135 - 137°C.

Example 7.

To a solution of 250 mg. of *tert*.-butoxycarbonyl-$N^G$-nitro-L-arginyl-L-methionine-β-naphthylamide which had been prepared in the same way as in Example 1 from *tert*.-butoxycarbonyl-$N^G$-nitro-L-arginine and L-methionine-β-naphthylamide in 30 mℓ. of absolute methanol were added 200 mg. of palladium-on-carbon and 0.2 mℓ. of $BF_3$-ether.

Into the mixture was bubbled hydrogen gas with stirring. When evolution of carbon dioxide was no longer observed, hydrogen gas was passed through for additional one hour (4 - 5 hours in total). The palladium-on-carbon was filtered off, and the filtrate was concentrated under reduced pressure. The residue thus obtained was purified by means of silica gel column chromatography. There was obtained 98 mg. of tert.-butoxycarbonyl-L-arginyl-L-methionine-β-naphthylamide. Yield 41 %. m.p. 106 - 107°C.

Example 8.

A solution of 250 mg. of tert.-butoxycarbonyl-D-leucyl-L-tyrosyl-L-methionine-β-naphthylamide which had been prepared in the same way as in Example 1 from L-tyrosyl-methionine-β-naphthylamide and tert.-butoxycarbonyl-D-leucine in 30 mℓ. of formic acid was treated in the same way as in Example 2 to obtain 200 mg. of D-leucyl-L-tyrosyl-L-methionine-β-naphthylamide formate. Yield 88 %. m.p. 223 - 225°C.

Example 9.

A solution of 400 mg. of tert.-butoxycarbonyl-β-alanyl-L-tyrosyl-L-methionine-β-naphthylamine prepared in the same way as in Example 1 from tert.-butoxycarbonyl-β-alanine and L-tyrosyl-L-methionine-β-naphthylamide in 50 mℓ. of formic acid was treated in the same way as in Example 2 to obtain 215 mg. of β-alanyl-L-tyrosyl-L-methionine-β-naphthylamide formate. Yield 60 %. m.p. 188 - 190°C.

Example 10.

The same procedures as in Example 1 were followed with 76 mg. of glycolic acid and 437 mg. of L-tyrosyl-L-methionine-β-naphthylamide followed by purification by silica gel column chromatography and subsequent crystallization from ethyl acetate-ether. There was obtained 218 mg. of glycoloyl-L-tyrosyl-L-methionine-β-naphthylamide. Yield 44 %. m.p. 251 - 253°C. (decomposed).

Example 11.

To a solution of 1.4 g. of L-tyrosyl-L-methionine-β-naphthylamide and 0.9 g. of triethylamine in 150 mℓ. of acetonitrile was dropwise added a solution of 0.36 g. of succinic anhydride in 80 mℓ. of acetonitrile with ice cooling and stirring. Stirring was continued for 30 min. with ice cooling and for additional 4 hours at room temperature. Then, the solvent was distilled off, and to the residue was added 50 mℓ. of water. The mixture was made acidic with 10 % aqueous citric acid, followed

by addition of ethyl acetate. The mixture was vigorously shaken, and the ethyl acetate layer was separated. The organic layer was dried over magnesium sulfate and then concentrated to dryness. Crude crystals thus obtained were recrystallized from methanol-water to obtain 1.34 g. of succinyl-L-tyrosyl-L-methionine-β-naphthylamide. Yield 83.2 %. m.p. 220 - 221°C.

Example 12.

The same procedures as in Example 1 were followed with 237 mg. of tert.-butoxycarbonyl-p-aminobenzoic acid and 437 mg. of L-tyrosyl-L-methionine-β-naphthylamide. The residue obtained was purified by means of silica gel column chromatography. There was obtained 400 mg. of tert.-butoxycarbonyl-p-aminobenzoyl-L-tyrosyl-L-methionine-β-naphthylamide, which was dissolved in 20 mℓ. of formic acid and then treated in the same way as in Example 2 to obtain 205 mg. of p-aminobenzoyl-L-tyrosyl-L-methionine-β-naphthylamide formate. Yield 55.8 %. m.p. 235 - 238°C. (decomposed).

Example 13.

A solution of 200 mg. of tert.-butoxycarbonyl-O-benzyl-D-seryl-L-tyrosyl-L-methionine-β-naphthylamide prepared in the same way as in Example 1 from tert.-

butoxycarbonyl-O-benzyl-D-serine and L-tyrosine-L-methionine-β-naphthylamide in 10 mℓ. of trifluoroacetic acid was stirred at room temperature for 2 hours and then concentrated under reduced pressure. The residue thus obtained was crystallized from methanol-ethanol-*n*-hexane to obtain 165 mg. of O-benzyl-D-seryl-L-tyrosyl-L-methionine-β-naphthylamide trifluoroacetate. Yield 92.2 %. m.p. 188 - 190°C.

Example 14.

A solution of 400 mg. of *tert*.-butoxycarbonyl-L-methionine-4-methylcoumaryl-7-amide in 5 mℓ. of trifluoroacetic acid was stirred at room temperature for 20 min., and the trifluoroacetic acid was distilled off. To the oily substance thus obtained was added 30 mℓ. of ether, and precipitates formed were separated by filtration. A solution of the precipitates and 375 mg. of *tert*.-butoxycarbonyl-L-tyrosyl-N-hydroxysuccinimide ester in 10 mℓ. of DMF was stirred at room temperature for 10 hours. To the resulting solution were added 200 mℓ. of ethyl acetate and 150 mℓ. of saturated aqueous sodium chloride, and the mixture was vigorously shaken. The ethyl acetate layer was separated, successively washed with 1N aqueous hydrochloric acid, saturated aqueous sodium bicarbonate and saturated aqueous sodium chloride, dried

over magnesium sulfate and concentrated to dryness. The residue thus obtained was crystallized from chloroform-n-hexane to obtain 300 mg. of tert.-butoxycarbonyl-L-tyrosyl-L-methionine-4-methylcoumaryl-7-amide. Yield 55.5 %. m.p. 211 - 212°C.

Example 15.

A solution of 220 mg. of tert.-butoxycarbonyl-L-tyrosyl-L-methionine-4-methylcoumaryl-7-amide in 2 mℓ. of trifluoroacetic acid was stirred at room temperature for 30 min., and the trifluoroacetic acid was distilled off. To the residue thus obtained was added 10 mℓ. of ether. White crystals, L-tyrosyl-L-methionine-4-methylcoumaryl-7-amide trifluoroacetate thus formed were separated by filtration. To a solution of the precipitates and 200 mg. of triethylamine in 3 mℓ. of acetonitrile and 3 mℓ. of tetrahydrofuran was dropwise added a solution of 46 mg. of succinic anhydride in 4 mℓ. of acetonitrile with ice cooling and stirring. The resulting mixture was stirred at room temperature for 3 hours and then allowed to stand overnight. The residue from distillation of the solvent was dissolved in 150 mℓ. of ethyl acetate. The ethyl acetate solution was successively washed with 1N aqueous hydrochloric acid and saturated aqueous sodium chloride, dried over magnesium

sulfate and concentrated to dryness. The residue thus obtained was crystallized from methanol-ethyl acetate-petroleum ether to obtain 100 mg. of succinyl-L-tyrosyl-L-methionine-4-methylcoumaryl-7-amide. Yield 45 %. m.p. 185 - 186°C.

Example 16.

A solution of 1.17 g. of _tert_.-butoxycarbonyl-L-tyrosyl-N-succinimide ester and 0.759 g. of L-methionine-_p_-nitroanilide in 50 mℓ. of tetrahydrofuran was stirred at room temperature for 10 hours and then concentrated under reduced pressure. To the concentrate were added 150 mℓ. of ethyl acetate and 100 mℓ. of water, and the mixture was vigorously shaken. The ethyl acetate layer was separated by filtration and successively washed with 1N aqueous hydrochloric acid, saturated aqueous sodium bicarbonate and saturated aqueous sodium chloride, dried over magnesium sulfate and concentrated to dryness. The residue thus obtained was crystallized from ethyl acetate-_n_-hexane to obtain 1.17 g. of _tert_.-butoxycarbonyl-L-tyrosyl-L-methionine-_p_-nitroanilide. Yield 78 %. m.p. 189 - 190°C.

Example 17.

A solution of 1.07 g. of _tert_.-butoxycarbonyl-L-tyrosyl-L-methionine-p-nitroanilide in 8 mℓ. of trifluoroacetic acid was stirred at room temperature for 30 min., and the trifluoroacetic acid was then distilled off. To the residue thus obtained was added 70 mℓ. of ether to form white crystals of L-tyrosyl-L-methionine-p-nitroanilide trifluoroacetate, which were separated by filtration. To a solution of the precipitates and 0.610 g. of triethylamine in 100 mℓ. of acetonitrile was dropwise added a solution of 0.221 g. of succinic anhydride in 30 mℓ. of acetonitrile with ice cooling and stirring. After allowed to stand overnight, the solvent was distilled off, and the residue was dissolved in 200 mℓ. of ethyl acetate. The solution was washed with 10 % aqueous citric acid, dried over magnesium sulfate and concentrated to dryness. The residue thus obtained was purified by silica gel column chromatography (chloroform: methanol = 10:1) and crystallized from dimethyl sulfoxide-water. There was obtained 0.15 g. of succinyl-L-tyrosyl-L-methionine-p-nitroanilide. Yield 47.7 %. m.p. 152 - 154°C.

CLAIMS

1. A peptide derivative having the general formula

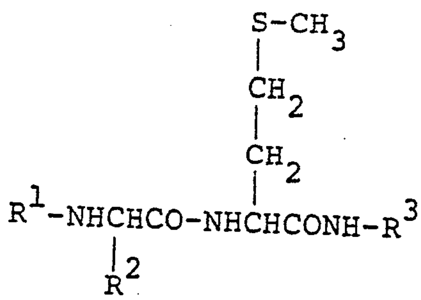

wherein $R^1$ represents an aliphatic acyl group containing 2 to 5 carbon atoms which substituted with one or two methyl, amino, carboxy, hydroxy or benzyloxy groups; a tertiary butoxycarbonyl group; an aminobenzoyl group; or a hydrogen atom; $R^2$ represents a 2-methylpropyl, 1-methylpropyl, p-hydroxybenzyl, p-methoxybenzyl, benzyl or 3-guanidylpropyl group; and $R^3$ represents a naphthyl, methylcoumaryl or nitrophenyl group; or an acid addition salt thereof.

2. A peptide derivative according to claim 1 wherein $R^1$ is a succinyl, leucyl or ß-alanyl group, $R^2$ is a p-hydroxybenzyl group and $R^3$ is a naphthyl group.

3. A peptide derivative according to claim 1 which is D-leucyl-L-tyrosyl-L-methionine-ß-naphthylamide, ß-alanyl-L-tyrosyl-L-methionine-ß-naphthylamide,

glycoloyl-L-tyrosyl-L-methionine-ß-naphthylamide, succinyl-L-tyrosyl-L-methionine-ß-naphthylamide, or succinyl-L-tyrosyl-L-methionine-p-nitroanilide.

4. An assay for a cysteine protease with a thiol group at the active site which assay comprises incubating the protease with a substrate and measuring changes in fluroescence or absorbance, characterized in that the substrate is a peptide derivative having the general formula

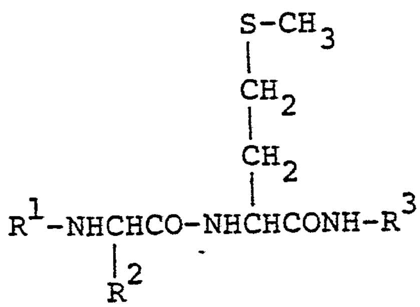

wherein $R^1$ represents an aliphatic acyl group containing 2-5 carbon atoms which is substituted with one or two methyl, amino, carboxyl hydroxy or benzyloxy groups; a tertiary butoxycarbonyl group; aminobenzoyl group; or a hydrogen atom; $R^2$ represents a 2-methylpropyl, 1-methylpropyl, p-hydroxybenzyl, p-methoxybenzyl, benzyl or 3-guanidylpropyl group; and $R^3$ represents a naphthyl, methylcoumaryl or nitrophenyl group; or an acid addition salt thereof.